(19) Europäisches Patentamt
European Patent Office
Office européen des brevets

(11) Veröffentlichungsnummer: **0 024 306**
**B1**

(12) # EUROPÄISCHE PATENTSCHRIFT

(45) Veröffentlichungstag der Patentschrift: **03.12.86**

(21) Anmeldenummer: **80104221.9**

(22) Anmeldetag: **18.07.80**

(51) Int. Cl.$^4$: **C 07 C 121/00,**
C 07 C 69/753, C 07 C 49/553,
C 11 B 9/00, A 23 L 1/226,
A 61 K 7/46

(54) **Neue substituierte Tetraline und Indane (I), Verwendung von (I) als Riech- und/oder Geschmackstoffe sowie Riech- und/oder Geschmackstoffkompositionen mit einem Gehalt an (I).**

(30) Priorität: **10.08.79 CH 7355/79**
**30.06.80 CH 5003/80**

(43) Veröffentlichungstag der Anmeldung:
**04.03.81 Patentblatt 81/09**

(45) Bekanntmachung des Hinweises auf die Patenterteilung:
**03.12.86 Patentblatt 86/49**

(84) Benannte Vertragsstaaten:
**CH DE FR GB LI NL**

(56) Entgegenhaltungen:
**CH-A- 338 819**
**DE-A-1 811 289**
**US-A-2 857 433**
**US-A-3 278 621**
**US-A-3 910 853**

**CHEMISCHE BERICHTE, Band 111, 1978, Verlag Chemie, GmbH. WEINHEIM (DE). H. A. STAAB et al.: "Bromwasser-stoff-Addition an 1,1,4,4-Tetra-methyl-1,4 -dihydronaphthalin: Synthesen von Ausgangs- und Reaktionsprodukten; Produktbilanz und sterischer Verlauf der Reaktion", Seiten 2965-2981**

(73) Patentinhaber: **L. Givaudan & Cie Société Anonyme**
**Patentdienst Postfach**
**CH-4002 Basel (CH)**

(72) Erfinder: **Gonzenbach, Hans Ulrich, Dr.**
**61 bis rue de Lyon**
**CH-1203 Genf (CH)**
Erfinder: **Ochsner, Paul Albert, Dr.**
**30 avenue des Tilleuls**
**CH-1203 Genf (CH)**

(74) Vertreter: **Urech, Peter, Dr. et al**
**Grenzacherstrasse 124 Postfach 601**
**CH-4002 Basel (CH)**

EP 0 024 306 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft Riech- und/oder Geschmackstoffkompositionen. Diese sind gekennzeichnet durch einen Gehalt an einer Verbindung der Formel

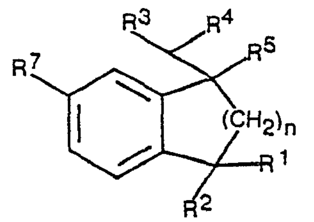

I

worin $R^1$ $-CO-CH_2-R^6$ darstellt, die Reste $R^2$ bis $R^7$ für Wasserstoff oder Methyl stehen und n = 1 oder 2 ist.

Die Formel soll demgemäss die Tetralinderivate Ia und die Indanderivate Ib repräsentieren:

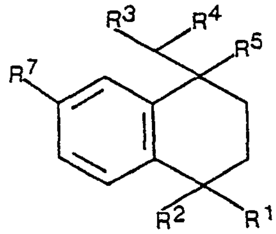

Ia

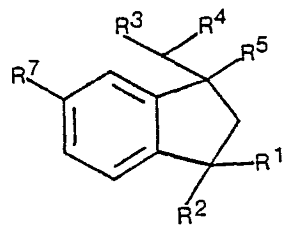

Ib

worin $R^1$ bis $R^7$ obige Bedeutung besitzen.

Die Verbindungen der Formel Ia sind bevorzugt.

Diese Verbindungen der Formel Ia sind neu. Die Verbindungen der Formel Ib sind, mit Ausnahmne des 1,1-Dimethyl-3-acetylindans, des 1,1,3-Trimethyl-3-acetylindans und des 1,1,3,6-Tetramethyl-3-acetylindans, ebenfalls neu. Die Erfindung betrifft demgemäss auch die neuen Verbindungen der Formel I.

Die Verbindungen I weisen besondere organoleptische Eigenschaften auf, auf Grund derer sie sich vorzüglich als Riech- und/oder Geschmackstoffe eignen.

Die Erfindung betrifft demgemäss auch die Verwendung der Verbindungen I als Riech- und/oder Geschmackstoffe.

Es sind zwar eine ganze Reihe von funktionalisierten Tetralinen und Indanen als Moschusriechstoffe bekanntgeworden. Sämtlichen dieser bekannten Körper ist als Strukturmerkmal gemeinsam, dass sie die funktionelle Gruppe am Benzolring tragen, siehe z.B. CH—PS 338,819 (Polak's Frutal Works Inc.). Die Verbindungen der Formel I sind andererseits am Cyclopentyl- oder Cyclohexylrest funktionalisiert.

Die Vebindungen der Formel I eignen sich aufgrund ihrer natürlichen Geruchsnoten und ihrer Haftdauer (Langzeiteffekt, insbesondere bezüglich Frische) insbesondere zur Modifizierung von bekannten, z.B.

α) blumigen Kompositionen, in denen z.B. die Citrusnoten verstärkt zum Ausdruck kommen sollen (z.B. für Cologne-Typen u.ä., Extraits),

β) des weiteren aber auch von fruchtigen, z.B. Typ Himbeere (Extrait-Typen, Komposition der femininen Richtung), von

γ) Tabak- und Holzkompositionen, (Extrait-Typen der masculinen Richtung) und schliesslich von

δ) Kompositionen mit grünen Noten, wo insbesondere eine erwünschte Abrundung und harmonisierende Effekte erzielt werden.

Besonders interessante Verbindungen sind:

1,1-Dimethyl-4-propionyl-teralin: sehr natürlich wirkende, ausserordentlich lang anhaltende Rosennote. Der Geruch erinnert an blonden Tabak, Trockenfrüchte, Honig, an die Damascone und an Givescone ® (s.u.). In Kompositionen dringen diese Noten zwar kaum durch. Dafür werden überraschende Effekte erzielt, die einerseits in einer natürlich wirkenden harmonischen Abrundung und gleichzeitigen Akzentuierung der Citrusnoten und andererseits in lang anhaltender Harmonie des Gemisches bestehen. Dies gilt besonders auch für die Citrusnoten. Die Kompositionen wirken eindeutig länger frisch als gleiche Kompositionen ohne Zusatz.

1,1-Dimethyl-4-acetyl-tetralin: nach Rosen, Tabak, Damasconen, fruchtig, honigartig.

Die Verbindungen I verbinden sich mit zahlreichen bekannten Riech- und/oder Geschmackstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

*Naturprodukte,* wie Baummoos-Absolue, Basilikumöl, Bergamottöl, acetyliertes Cedernholzöl (z.B. Vertofix® IFF bzw. Cedartone® Givaudan), Eichenmoos, Galbanumöl, Geraniumöl, Jasmin Absolue und seine Substitute, Lavendelöl, Lavandinöl, MastixAbsolue, Neroliöl, Patchouliöl, Petitgrainöl Paraguay, Sandelholzöl, Vetiveröl, Ylang-Ylang-Oel, Zitronenöl, Wermutöl,

*Alkohole,* wie Linalool, Cintronellol, Geraniol, natürliches Rhodinol, α-Terpineol, Phenyläthylalkohol, Phenylpropylalkohol, Zimtalkohol, 3-Methyl-5-(2′,2′,3′-trimethyl-cyclopent-3′-en-1′-yl)-pentan-2-ol (Sandalore® Givaudan).

*Aldehyde,* wie 3,5-Dimethyl-cyclohex-3-en-carboxaldehyd, Decanal, Methylnonylacetataldehyd, Hydroxycitronellal, α-Hexylzimtaldehyd, Cyclamenaldehyd, p-tert. Butyl-α-methyl-dihydro-zimt-aldehyd (z.B. Lilial® Givaudan), Citral,

*Ketone,* wie α-Jonon, Acetylcedren, p-Methylacetophenon, Methyljonone,

*Ester,* wie Cedrylacetat, cis-3-Hexenylacetat, cis-3-Hexenylbenzoat, Aethylacetoacetat, Linalylacetat, Geranylacetat, Terpenylacetat, Phenyläthylacetat, Styrallylacetat, p-tert. Butylcyclohexylacetat, 4[4-Methyl-3-pentenyl-]-cyclohex-3-en-1-yl-carbinylacetat (z.B. Myraldylacetat® Givaudan), Cinnamylformiat, Benzylacetat, Benzylsalicylat, Amylsalicylat, Methyldihydrojasmonat, 3-Aethyl-1,1-dimethyl-cyclohex-3-en-2-carbonsäureäthylester (Givescone® Givaudan),

*Lactone,* wie γ-Undecalacton, Cumarin,

*verschiedene weitere, in der Parfümerie oft benützten Komponenten* wie Moschus-Verbindungen (Ambrette-Ketonmoschus, 12-Oxa-hexadecanolid (z.B. Musk 174® Naarden), 1,1-Dimethyl-4-acetyl-6-tert.butylindan, Indol), p-Menthan-8-thiol-3-on, Eugenol, Acetaldehyd-propylphenyl-äthylacetal, Methyl-1-methyl-cyclododecyläther (z.B. Madrox® Givaudan).

Die Verbindungen der Formel I lassen sich in weiten Grenzen einsetzen, die beispielsweise von 0,1 (Detergentien) −50% (alkoholische Lösungen) in Kompositionen reichen können, ohne dass diese Werte jedoch Grenzwerte darstellen sollen, da der erfahrene Parfümeur auch mit noch geringeren Konzentration Effekte erzielen oder aber mit noch höheren Dosierungen neuartige Komplexe aufbauen kann. Die bevorzugten Konzentrationen bewegen sich zwischen 0,5 und 25%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und — wie obige Zusammenstellung zeigt — unter Verwendung einer breiten Palette bekannter Riechstoffe, verwendet werden. Bei der Herstellung solcher Kompositionen können die oben aufgeführten bekannten Riechstoffe nach (dem Parfümer bekannter) Art und Weise verwendet werden, wie z.B. aus W. A. Poucher, Perfumes, Cosmetics and Soaps *2*, 7. Auflage, Chapman und Hall, London, 1974 hervorgehend.

Als Geschmackstoffe können die Verbindungen I beispielsweise zur Erzeugung bzw. Verbesserung, Verstärkung, Steigerung oder Modifizierung von Frucht- oder Beerenaromen, insbesondere Himbeeraromen, in Nahrungsmitteln (Joghurt, Süsswaren, etc.); in Genussmitteln (Tee, Tabak, etc.) und Getränken (Limonaden etc.) verwendent werden.

Die ausgeprägten geschmacklichen Qualitäten der Verbindungen I ermöglichen die Verwendung in geringen Kozentrationen. Eine geeignete Dosierung umfasst beispielsweise den Bereich von 0,01 ppm—100 ppm, vorzugsweise von 0,1 ppm—20 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50—500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1—10, insbesondere 0,5—3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC—Press, Inc., Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Monoatriumglutamat, Dinatriuminosin-5′-monophosphat (IMP), Dinatriumguanosin-5-phosphat (GMP); oder spezielle Aromastoffe, Wasser, Aethanol, Propylenglykol, Glycerin.

Die Verbindungen der Formel I sind beispielsweise gemäss Reaktionsschema I zugänglich. Bei den dort aufgeführten Methoden handelt es sich um an sich bekannte Verfahren; es sind dies insbesondere (siehe auch Tabelle I):

ⓐ Alkenylierungen

ⓑ Alkylierungen (Methylierungen)

ⓒ Acylierungen

ⓓ Cyclisierungen
ⓔ — ⓖ Hydrolysen
ⓗ — ⓚ Ketonsynthesen
ⓛ Herstellung von reaktiven Säurederivaten
○— ⓞ Herstellung der Ester

Die Tabelle I informiert über zweckmässige Methoden und Mittel sowie zweckmässige und bevorzugte Reaktionsparameter der einzelnen Verfahrensvarianten.

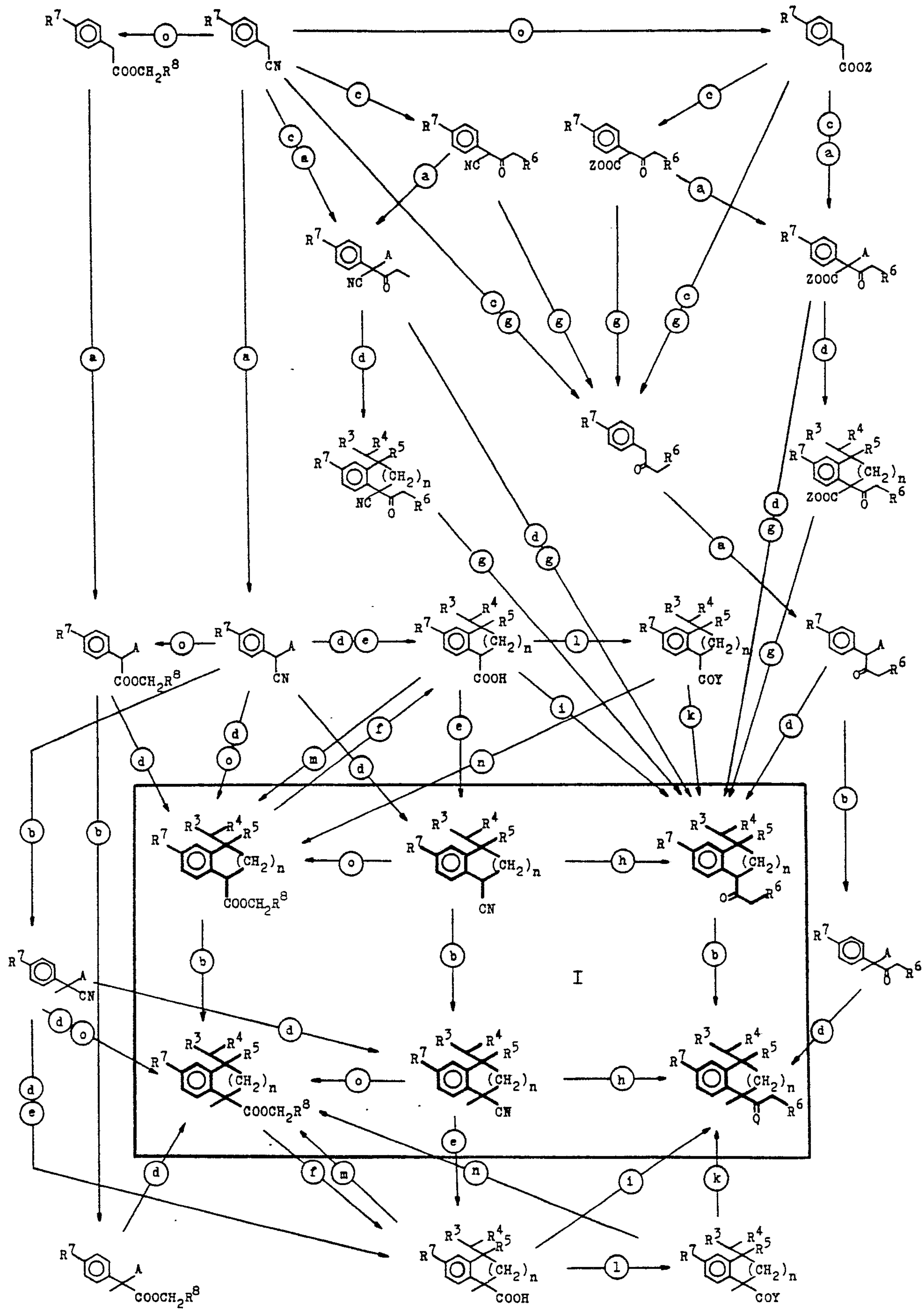

**Tabelle I**

| Reaktion | | Edukt | Produkt | Reagens | Reaktionsbedingungen Lsm/Kat./etc. | Bemerkungen / Literatur |
|---|---|---|---|---|---|---|
| ⓐ Alkenylierung | | 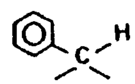 | 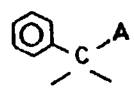 | XA } XA' } | a) unter Phasentransferbed. | Jozef Dockx, Synthesis 1973, 441 |
| ⓑ Methylierung | | 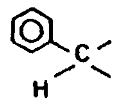 | 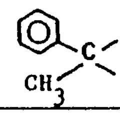 | $XCH_3$ | b) klassisch z.B. +OH/+OK EtOH/EtONa $Et_2O$/NaH etc. | ORGANIKUM, VEB, Deutscher Verlag der Wissenschaften, Berlin, 1967 |
| ⓒ Acylierung | | 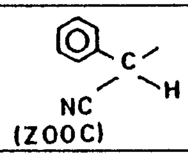 | 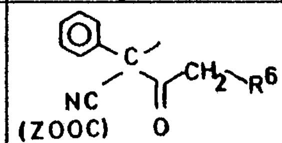 | $Y-COCH_2R^6$ (bevorzugt Ester) | z.B. EtOH/ EtONa $Et_2O$/NaH etc. | H.O.House, "Modern Synthetic Reactions" W.A. Benjamin, Inc., New York, Amsterdam 1965 |
| ⓓ Cyclisierung | | 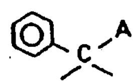 | 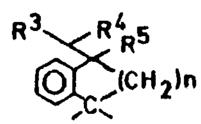 | PPS } $H_3PO_4$ 85% } | "mild" 50-100°C | Cyclisierung gelingt unter Erhaltung der Nitrilfunktion, im Gegensatz zu E. GROCHOWSKI + T. BOLESLAWSKA Bull. Acad. Polon. Sci., Ser. Sci. Chim. 20, 297 (1972) |
| ⓔ Hydrolyse von | Nitril | 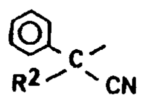 | 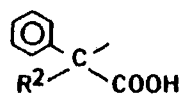 | Mineralsäuren (bevorzugt), wässrige Laugen | z.B. $H_2SO_4$ 60% 100°C | Unter diesen Bedingungen cyclisieren Verbindungen vom Typ 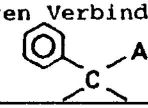 gleichzeitig |
| ⓕ | Ester | 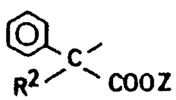 | | Mineralsäuren, wässrige Laugen (bevorzugt) | z.B. { KOH $H_2O$ EtOH } | |

| Reaktion | | Edukt | Produkt | Reagens | | Reaktionsbedingungen Lsm/Kat./etc. | Bemerkungen/Literatur |
|---|---|---|---|---|---|---|---|
| (g) Hydrolyse von | Keto-nitril (Keto-ester) gefolgt von De-carboxy-lierung | 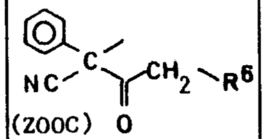 | 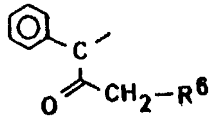 | sec. Nitril (Ester) | Mineral-säuren, Carbon-säuren | z.B. $H_2SO_4$ 60% | Organic Synthesis, Coll. Vol. IV, 174 (1963) |
| | | | | tert. Nitril (Ester) | starke Säuren: | "energisch", z.B. $H_2SO_4$ konz. o. $H_3PO_4$ 100%, HBr 48%, Eisessig 100-150°C | Sterische Hinderung in tert. Ni-trilen (Estern) verlangt energische Bedingungen, unter welchen Verbin-dungen vom Typ 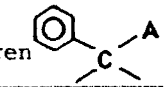 gleichzeitig cyclisieren |
| Ketonsynthesen aus: | (h) Nitril (i) freier Säure (k) reakt. Säure-derivat | 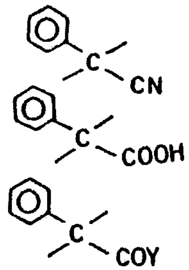 | 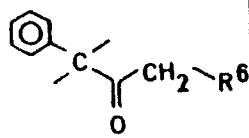 | $MCH_2R^6$ | | $CH_3MgX$ | ibid. Seite 603 → Allg. F. Wingler, HOUBEN-WEYL 7/2a, 545 (1973) |
| | | | | $LiCH_2R^6$ | | insbes. $LiCH_3$ | M.J. Jorgenson, Org. Reactions 18, 1 |
| | | | | $MCH_2R^6$ | | $C_2H_5MgBr$ CuI als Kata-lysator Y = Cl | J.E. Dubois, M. Boussu, C. Lion THL 1971, 829. |
| (l) Herstellung des reakt. Säurederivates | | 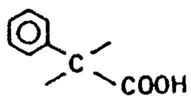 | 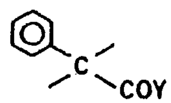 (Y = Cl bevorzugt) | allg. → | | | ORGANIKUM/HOUBEN-WEYL |
| | | | | z.B. $PCl_3$ | | 20-50°C ohne Lsm | ORGANIKUM/HOUBEN-WEYL |

Herstellung der Ester aus:

| Reaktion | Edukt | Produkt | Reagens | Reaktionsbedingungen Lsm/Kat./etc. | Bemerkungen/Literatur |
|---|---|---|---|---|---|
| (m) freier Säure | 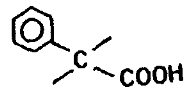 | 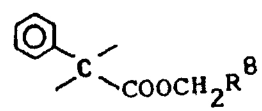 | allg. | | ORGANIKUM/HOUBEN-WEYL |
| (n) reakt. Säurederivaten | 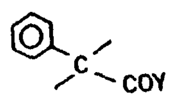 (Y=Cl bevorzugt) | | allg. | | ORGANIKUM/HOUBEN-WEYL |
| (o) Nitrilen | 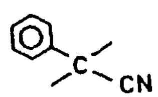 | | ZOH/ $R^8CH_2OH$ Mineralsäure | $EtOH/H_2SO_4$ Siedetemp. | Org. Synth. Coll, Vol I, 270 (1958). |

Legende zu Tabelle I und Schema I

A = 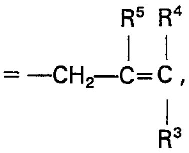 , 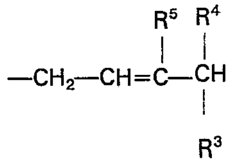 , 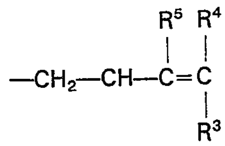

A' = 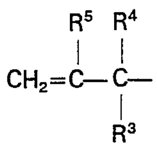 , 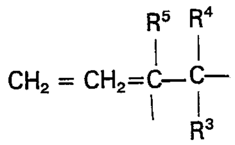 ,

X = Halogen (—Cl, —Br, —J)

Y = —X ,, (↑ Säure-halogenid) —OZ , (↑ Ester) —OCOZ , (↑ gemischtes Anhydrid) $—OCOCH_2R^6$ (↑ sym. Anhydrid) $—NH_2$ (↑ Amid)

Z = Niederalkyl
PPS = Polyphosphorsäure
M = organisch gebundenes Metall, also z.B.

$—Li, —MgX, —CdCH_2R^6, —Al(CH_2R^6)_2 —MnJ, —ZnCH_2R^6$

ⓐ oder ⓑ vorteilhaft an ⓒ anschliessen, wobei Zusatz von Base überflüssig; das intermediär gebildete Natrium — (Kalium —, Lithium — etc.) Salz reagiert mit XA/XA'
ⓖ vorzugsweise an ⓒ anschliessend
ⓓ + ⓔ vorzugsweise in einem Schritt
ⓓ + ⓞ ,, ,, ,, ,,

*es bedeuten*

.... } einzeln oder Gemisch
....

{.... } Gemisch
 ....

Lsm = Lösungsmittel

Herstellung der Ausgangsmaterialien:

A) 5-Methyl-2-phenyl-4-hexennitril

In einem 2-Liter-Kolben, versehen mit Thermometer, Rührer und Rückflusskühler werden 384 g 50%ige Natronlauge (4,8 Mol) und 12 g Hexadecyl-trimethylammoniumbromid vorgelegt und unter Rühren innert einer Viertelstunde ein Gemisch von 469 g Benzylcyanid (4 Mol) und 502 g Prenylchlorid[1]) (4,8 Mol) zugetropft; während der Zugabe erwärmt sich das Gemisch, es wird anschliessend noch während 3 Stunden bei 60°C weitergerührt. Nach Abkühlung wird auf 500 g Eis gegossen, mit Aether ausgeschüttelt (4 ×) und mit Wasser neutral gewaschen. Die Phasentrennung kann durch die Zugabe von wenig Alkohol erleichtert werden. Nach Trocknung über Natriumsulfat wird der Aether abgedampft und fraktioniert destilliert. Man erhält 444 g 5-Methyl-2-phenyl-4-hexennitril, Sdp. 108—109°C/3 mmHg, $n_D^{20}$ : 1,5171—72, Ausbeute 60%.

1) Gemisch von 1-Chlor-3-methyl-2-buten und
3-Chlor-3-methyl-1-buten

B) 4-methyl-2-phenyl-4-pentennitril
Wird in obigem Verfahren anstelle von Prenylchlorid β-Methallychlorid verwendet, erhält man 4-Methyl-2-phenyl-4-pentennitril, Sdp. 72°C/0,13 mmHg, $n_D^{20}$ : 1,5203

C) 5-Methyl-2-(p-tolyl)-4-hexennitril
Aus p-Methyl-benzylcyanid und Prenylchlorid erhält man in analoger Weise 5-Methyl-2-(p-tolyl)-4-hexennitril, Sdp. 89°C/0,03 mmHg, $n_D^{20}$ : 1,5176.

D) Aethyl-benzyl-keton
In einem 2-Liter-Kolben, versehen mit Thermometer, Rührer, Tropftrichter und Rückflusskühler werden 600 ml absoluten Alkohols unter Rühren portionenweise mit 30 g Natriummetall versetzt und bis zur Auflösung des Metalls bei Rückflusstemperatur gehalten. Innert 1/2 Stunde wird nun zur siedenden Lösung ein Gemisch von 117,2 g Benzylcyanid und 153,2 g Propionsäure-äthylester zugetropft und während 3 Stunden weitererhitzt. Darauf wird der Alkohol abdestilliert und es werden 300 ml Toluol, gefolgt von 250 ml Eiswasser, zugegeben. Die wässrige Phase wird abgetrennt, nochmals mit 300 ml Toluol gewaschen und hierauf werden 540 g 96%ige Schwefelsäure zugegeben. Man hält bei Rückflusstemperatur, bis die $CO_2$—Entwicklung aufhört, kühlt ab, verdünnt mit 250 ml Eiswasser und extrahiert mix 2 × 300 ml Toluol. Es folgen Waschen mit Wasser, mit 10%iger Natriumcarbonatlösung und erneut mit Wasser, Trocknen über Natriumsulfat und Eindampfen. Die Destillation ergibt 79 g Aethyl-benzyl-keton, Sdp. 69°C/3 mmHg, $n_D^{20}$ : 1,5109, Ausbeute 53%.

E) 2-Methyl-5-phenyl-oct-2-en-6-on
In einem 2-Liter-Kolben, versehen mit Thermometer, Rührer und Rückflusskühler werden 192 g 50%ige Natronlauge (2.4 Mol) und 6 g Hexadecyl-trimethylammoniumbromid vorgelegt, hierauf wird unter Rühren innert 1/2 Stunde ein Gemisch von 296 g Aethyl-benzyl-keton (2 Mol) und 251 g Prenylchlorid (2,4 Mol) zugetropft. Darauf wird auf 60°C erwärmt und während 3 Stunden gerührt. Man lässt abkühlen und gibt 500 ml Eiswasser zu, schüttelt viermal mit Aether aus und wäscht mit Wasser neutral. Die Phasentrennung kann durch Zugabe von wenig Alkohol erleichtert werden. Nach Trocknen über Natriumsulfat wird der Aether abgedampft und es folgt eine fraktionierte Destillation. Man erhält so 244 g 2-Methyl-5-phenyl-oct-2-en-6-on, Sdp. 106°C/3 mmHg, $n_D^{20}$ : 1,5092-88, Ausbeute 75%.

F) 2-Methyl-5-phenyl-hept-2-en-6-on
Wird in obigem Beispiel Aethyl-benzyl-keton durch Methyl-benzyl-keton ersetzt, so erhält man 2-Methyl-5-phenyl-hept-2-en-6-on, Sdp. 97°C/2 mmHg, $n_D^{20}$ : 1,5135.

G) 2-Methyl-4-phenyl-hept-1-en-5-on
Wird Aethyl-benzyl-keton mit β-Methallylchlorid entsprechend obigen Angaben umgesetzt, so erhält man 2-Methyl-4-phenyl-hept-1-en-5-on, Sdp. 93°C/3 mmHg, $n_D^{20}$ : 1,5094.

H) 2-methyl-4-phenyl-hex-1-en-5-on
Die Umsetzung von Methyl-benzyl-keton mit β-Methallylchlorid gemäss vorstehendem Verfahren liefert 2-Methyl-4-phenyl-hex-1-en-5-on, Sdp. 85°C/3 mmHg, $n_D^{20}$ : 1.5151.

J) 5-Phenyl-oct-2-en-6-on
Entsprechend obigen Angaben wird Aethyl-benzyl-keton mit Crotyl-chlorid umgesetzt und 5-Phenyl-oct-2-en-6-on erhalten, Sdp. 95°C/3 mmHg, $n_D^{20}$ : 1,5102.

J bis) 5-Phenyl-hept-2-en-6-on
Entsprechend den obigen Angaben wird Methyl-benzyl-keton mit Crotylchlorid oder 3-Chlor-1-buten umgesetzt und 5-Phenyl-hept-2-en-6-on erhalten, Sdp. 88°C/3 mmHg, $n_D^{20}$ : 1,5190. Bei Verwendung von 3-Chlor-1-buten entsteht ebenfalls 3-Methyl-5-phenyl-hex-1-en-5-on, das destillativ abgetrennt wird.

K) 5-Phenyl-oct-1-en-6-on
Entsprechend vorstehender Angaben wird Aethyl-benzyl-keton mit 1-Brom-3-buten umgesetzt und 5-Phenyl-oct-1-en-6-on erhalten, Sdp. 106°C/4 mmHg, $n_D^{20}$ : 1,5088.

K bis) 2-methyl-4-phenyl-but-1-en-4-säuremethylester
In einem 1-Liter-Kolben, versehen mit Thermometer, Rührer, Rückflusskühler und Tropftrichter werden 24 g Natriumhydrid in 400 ml absolutem Tetrahydrofuran vorgelegt und auf Rückflusstemperatur (70°C) erhitzt. Hierauf wird ein Gemisch von 152 g Phenylessigsäuremethylester und 91 g β-Methallychlorid langsam zugetropft (1 ½ Stunden). Nach Beendigung der Zugabe wird weitere 2 Stunden erhitzt und dann abkühlen gelassen. Es wird wenig Methanol zugegeben und hierauf auf 800 ml Wasser gegossen, mit Aether extrahiert, neutral gewaschen, getrocknet und eingedampft. Die Destillation ergibt 101 g 2-Methyl-4-phenyl-but-1-en-4-säuremethylester, Sdp. 90°C/4 mmHg, $n_D^{20}$ : 1,5078. Ausbeute 50%. Der Destillationsrückstand enthält Dibenzylketon.

L) 4-Cyano-7-methyl-4-phenyl-oct-6-en-3-on

In einen 1 ½-Liter-Kolben, versehen mit Thermometer, Rührer, Tropftrichter und Destillationsaufsatz werden 300 ml Dimethylformamid und 84 g Natriumalkoholat eingebracht und es wird unter Rühren innert ¾ Stunden ein Gemisch on 137 g Benzylcyanid und 143 g Propionsaüreäthylester zugetropft. Die Lösung erwärmt sich unter Rotfärbung auf etwa 50°C. Der gebildete Alkohol wird bei schwachem Vakuum abdestilliert und die Reaktionslösung auf Raumtemperatur Abkühlen gelasen, worauf 146 g Prenylchlorid in 140 ml Dimethylformamid zugegeben werden. Man rührt bei 60—65°C während 2 ½ Stunden, bzw. bis die Reaktionslösung neutrale Reaktion zeigt. Man kühlt ab, filtriert, wäscht mit Hexan nach und dampft ein. Der Rückstand wird in Hexan aufgenommen, mit Wasser gewaschen, über Natriumsulfat getrocknet, eingedampt und destilliert. Man erhält 150 g 4-Cyano-7-methyl-4-phenyl-6-en-3-on, Sdp. 95°C/0,08 mmHg, $n_D^{20}$ : 1,5160, Ausbeute 53%.

M) 4-Methoxycarbonyl-7-methyl-4-phenyl-oct-6-en-3-on

Analog vorstehendem Verfahren kann durch Ersatz von Benzylcyanid durch Phenylessigsäuremethylester 4-Methoxy-carbonyl-7-methyl-4-phenyl-oct-6-en-3-on gewonnen werden, Sdp. 108°C/0,08 mmHg, $n_D^{20}$ : 1,5202.

N) 1,1-Dimethyl-tetralin-4-carbonsäure

In einem 3-Liter-Kolben, versehen mit Thermometer, Rührer und Rückflusskühler legt man 1250 g 60%ige Schwefelsäure vor, erhitzt auf 60—70°C und tropft 463 g 5-Methyl-2-phenyl-4-hexennitril zu. Nach beendeter Wärmeentwicklung wird während 8 Stunden bei Rückflusstemperatur gehalten. Nach 15 Stunden wird das Reaktionsgemisch viermal mit Aether extrahiert. Man wäscht die organischen. Phasen mit Wasser, trocknet über Natriumsulfat und dampft ein. Man erhält 501 g Rohprodukt, die aus 625 ml Hexan umkristallisiert werden. Man gewinnt so 450 g 1,1-Dimethyl-tetralin-4-carbonsäure, Smp. 80—81°C, Ausbeute 88%.

O) 1,1,7-Trimethyl-tetralin-4-carbonsäure

Behandlung von 5-Methyl-2-(p-tolyl)-4-hexennitril entsprechend N liefert 1,1,7-Trimethyl-tetralin-4-carbonsäure, Smp. 103°C.

P) 1,1-Dimethyl-tetralin-4-carbonsäurechlorid

102 g 1,1-Dimethyl-tetralin-4-carbonsäure werden unter Rühren mit 27,4 g Phosphortrichlorid versetzt und bei 50°C 3 Stunden gerührt. Es wird dabei unter Feuchtigkeitsausschluss und Ableiten des entstehenden Salzsäuregases gearbeitet. Man trennt die obere Phase nach 12 Stunden ab und destilliert über eine kurze Vigreux-Kolonne. Man erhält 87 g 1,1-Dimethyl-tetralin-4-carbonsäurechlorid, Sdp. 89°C/0,2 mmHg, $n_D^{20}$ : 1,5418—20, Ausbeute 78%.

Q) 1,1,7-Trimethyl-tetralin-4-carbonsäurechlorid

Aus 1,1,7-Trimethyl-tetralin-4-carbonsäure wird entsprechend 1,1,7-Trimethyl-tetralin-4-carbonsäurechlorid erhalten, Sdp. 109°C/0,5 mmHg, $n_D^{20}$ : 1,5418.

R) 1,1-Dimethyl-4-cyano-tetralin

46,3 g 85%ige Phosphorsäure, 46,3 g Polyphosphorsäure, 250 ml Toluol und 92,6 g 5-Methyl-2-phenyl-4-hexennitril werden zusammen gegeben und unter Rühren während 3 Stunden auf 100°C erwärmt; das Gemisch wird hierauf auf Eis gegossen. Es wird nun mit Aether extrahiert, mit Wasser, mit 10%iger Natriumcarbonatlösung und erneut mit Wasser bis zum Neutralpunkt gewaschen, getrocknet, eingedampft und im Vakuum destilliert. Es werden 85,3 g 1,1-Dimethyl-4-cyano-tetralin erhalten, Sdp. 114°C/2 mm, $n_D^{20}$ : 1,5351. Ausbeute 92%. Geruch: angenehm fruchtig, tabakartig, nach Stroh mit Anklang an Pathouli und Ambra.

Beispiel 1

1) 1,1-Dimethyl-4-propionyl-tetralin

Zu einer aus 600 ml Aether, 34,7 g Magnesiumspänen und 166 g Aethylbromid hergestellten Grignard-Lösung werden 61,8 g 1,1-Dimethyl-4-cyano-tetralin in 100 ml absolutem Aether unter Rühren, zugetropft. Danach wird das Gemisch 6 Stunden bei Rückflusstemperatur gehalten, abgekühlt, mit 10%iger Salzsäure angesäuert, die wässrige Phase raschmöglichst abgetrennt und 1 Stunde bei Rückflusstemperatur gehalten. Man kühlt ab, extrahiert mit Aether, wäscht mit Wasser netural, trocknet über Natriumsulfat und dampft ein. Eine Destillation im Vakuum ergibt 39,4 g 1,1-Dimethyl-4-propionyl-tetralin, Sdp. 75°C/0,05 mmHg, $n_D^{20}$ : 1,5263, Ausbeute 55%. Geruch: Rosennote, tabakartig (blond), Trockenfrüchte, Beeren, Damascone, Honig.

2) 1,1-Dimethyl-4-propionyl-tetralin

36 g 85%ige Phosphorsäure, 36 g Polyphosphorsäure, 175 ml Toluol und 72 g 2-Methyl-5-phenyl-oct-2-en-6-on werden zusammen unter Stickstoffatmosphäre und unter Rühren während 2 Stunden auf 100°C

erwärmt. Man lässt abkühlen, fügt 250 ml Eiswasser hinzu, trennt die wässrige Phase ab und unterwirft die organische Phase einer Wasserdampfdestillation. Das Destillat wird vom Wasser abgetrennt und im Vakuum destilliert. Man erhält 50 g 1,1-Dimethyl-4-propionyl-tetralin, Sdp. 75°C/0,05 mmHg, $n_D^{20}$ : 1,5263, Ausbeute 70%. Geruch: siehe 1).

3) 1,1-Dimethyl-4-acetyl-tetralin
Obige Umsetzung von 2-Methyl-5-phenyl-hept-2-en-6-on liefert 1,1-Dimethyl-4-acetyl-teralin, Sdp. 69°C/0,07 mmHg, $n_D^{20}$ : 1,5333. Geruch: nach Rosen, Tabak, Damascone, fruchtig, honigartig.

4) 1-Methyl-4-propionyl-tetralin
5-Phenyl-oct-2-en-6-on oder 5-Phenyl-oct-1-en-6-on ergeben bei analoger Umsetzung 1-Methyl-4-propionyl-tetralin, Sdp. 79°C/0,02 mmHg, $n_D^{20}$ : 1,5333. Geruch: fruchtig, etwas minizig.

4 bis) 1-Methyl-4-acetyl-tetralin

5-Phenyl-hept-2-en-6-on ergibt bei analoger Umsetzung 1-methyl-4-tetralin, Sdp. 99°C/4 mmHg, $n_D^{20}$ : 1,5379. Geruch: Methyleugenol, Stroh.

5) 1,1-Dimethyl-3-propionyl-indan
2-Methyl-4-phenyl-hept-1-en-5-on liefert unter den angegebenen Bedingungen 1,1-Dimethyl-3-propionyl-indan, Sdp. 64°C/0,03 mmHg, $n_D^{20}$ : 1,5199. Geruch: wie 1) aber etwas schwächer.

6) 1,1-Dimethyl-3-acetyl-indan
2-Methyl-4-phenyl-hex-1-en-5-on liefert unter analogen Bedingungen 1,1-Dimethyl-3-acetyl-indan, Sdp. 82°C/0,3 mmHg, $n_D^{20}$ : 1,5249. Geruch: wie 1) mit zusätzlicher Holznote.

7) 1,1-Dimethyl-4-propionyl-tetralin
In einem 3-Liter-Kolben, versehen mit Thermometer und Rührer, werden unter Kühlen 200 g Phosphorpentoxid zu 500 g 85%iger Phosphorsäure gegeben und anschliessend bei ca. 50°C 150 g 4-Cyano-7-methyl-4-phenyl-oct-6-en-3-on zugegeben. Man rührt bei 105°C bis kein Ausgangsmaterial mehr vorhanden ist (GC/Dünnschicht). Darauf werden 1000 g 48%ige Bromwasserstoffsäure und 500 g Eisessig zugegeben. Nach 12 Stunden fügt man weitere 200 g 40%ige Bromwasserstoffsäure und 100 g Eisessig hinzu und erhöht die Temperatur auf 110°C. Nach total 30 Stunden Reaktionszeit wird abgekühlt, Eis zugegeben und mit Aether extrahiert, mit Wasser, mit 10%iger Natronlauge, wiederum mit Wasser neutral gewaschen, über Natriumsulfat getrocknet und eingedampft. Eine Vakuumdestillation ergibt 32 g 1,1-Dimethyl-4-propionyl-tetralin, Sdp. 75°C/0,05 mmHg, $n_D^{20}$ : 1,5263, Ausbeute 24%. Geruch: siehe 1).

8) 1,1-Dimethyl-4-propionyl-tetralin
Obige Umsetzung kann analog auch auf 4-Methoxycarbonyl-7-methyl-4-phenyl-oct-6-en-3-on angewandt werden und liefert so 1,1-Dimethyl-4-propionyl-tetralin, Sdp. 75°C/0,05 mmHg, $n_D^{20}$ : 1,5263. Geruch: siehe 1).

9) 1,1-Dimethyl-4-acetyl-tetralin
16,8 g 1,1-Dimethyl-tetralin-4-carbonsäure werden in 200 ml absolutem Aether unter Stickstoff- oder Argonatmosphäre tropfenweise mit 99,2 ml einer 2 molaren Lösung von Methyllithium in Aether (198,4 mMol) versetzt; es wird 1 Stunde bei Raumtemperatur gerührt und anschliessend noch 1 Stunde bei Rückflusstemperatur gehalten. Man giesst auf gesättigte Ammoniumchloridlösung, schüttelt mit Aether aus, wäscht mit Wasser neutral, trocknet über Natriumsulfat, dampft den Aether ab und destilliert fraktioniert. Man erhält 8,4 g 1,1-Dimethyl-4-acetyl-tetralin, Sdp. 69°C/0,07 mmHg, $n_D^{20}$ : 1,5333, Ausbeute 50%. Geruch: siehe 3).

10) 1,1-Dimethyl-4-propionyl-tetralin
In einem 1-Liter-Kolben, versehen mit Thermometer, Rührer, Tropftrichter und Rückflusskühler legt man 223 g 1,1-Dimethyl-tetralin-4-carbonsäurechlorid (1 Mol) und 11 g Kupfer(I)jodid vor und lässt aus dem Tropftrichter bei −10 bis −5°C eine frisch aus 27 g Magnesiumspänen (1,1 Mol) und 122 g Aethylbromid (1,12 Mol) in 500 ml absolutem Aether hergestellte Grigard-Lösung zutropfen. Es muss dabei mit etwas Aether verdünnt werden. Es wird 5 Stunden unter Kühlen weitergerührt, dann die Kühlung entfernt. Nachdem das Reaktionsgemisch Raumtemperatur erreicht hat, wird auf gekühlte, gesättigte Ammoniumchloridlösung gegossen, mit Aether ausgeschüttelt, mit Wasser neutral gewaschen, über Natriumsulfat getrocknet und eingedampft. Die fraktionierte Destillation ergibt 138 g 1,1-Dimethyl-4-propionyl-tetralin, Sdp. 75°C/0,05 mmHg, $n_D^{20}$ : 1,5263, Ausbeute 64%. Belüftung kann die olfaktische Qualität noch verbessern. Geruch: siehe 1).

11) 1,1-Dimethyl-4-acetyl-tetralin
Wird in obigem Verfahren die entsprechende Grignardlösung aus Methyljodid hergestellt, so erhält man 1,1-Dimethyl-4-acetyl-tetralin, Sdp. 69°C/0,07 mmHg, $n_D^{20}$ : 1,5333. Geruch: siehe 3).

12) 1,1,7-Trimethyl-4-propionyl-tetralin
Durch entsprechende Umsetzung wird aus 1,1,7-Trimethyl-tetralin-4-carbonsäurechlorid 1,1,7-Trimethyl-4-propionyl-tetralin erhalten, Sdp. 90°C/0,08 mmHg, $n_D^{20}$ : 1,5270. Geruch: fruchtig, Himbeeren.

13) 1,1,7-Trimethyl-4-acetyl-tetralin
Durch entsprechende Umsetzung wird aus 1,1,7-Trimethyl-tetralin-4-carbonsäurechlorid 1,1,7-Trimethyl-4-acetyl-tetralin erhalten, Sdp. 73°°C/0,02 mmHg, $n_D^{20}$ : 1,5309. Geruch: fruchtig, jononartig.

14) 1,1,4-Trimethyl-4-propionyl-tetralin
5,6 g Kalium-tert.butylat werden in 42,5 ml tert.-Butanol vorgelegt und dazu 10,8 g 1,1-Dimethyl-4-propionyl-tetralin getropft. Es wird 1 Stunde gerührt, auf 5°C abgekühlt und 8,5 g Methyljodid zugegeben. Man rührt 3 Stunden weiter, gibt weitere 4,2 g Methyljodid hinzu, entfernt die Kühlung und rührt weitere 15 Stunden. Danach wird die Hauptmenge des tert.-Butanols abgedampft, der Rückstand in Aether aufgenommen, mit Wasser neutral gewashen, über Natriumsulfat getrocknet, eingedampft und destilliert. Man erhält 6 g 1,1,4-Trimethyl-4-propionyltetralin, Sdp. 79°C/0,05 mmHg, $n_D^{20}$ : 1,5249, Ausbeute 52 %. Geruch: fruchtig, holzig.

15) 1,1,4-Trimethyl-4-acetyl-tetralin
Durch entsprechende Methylierung von 1,1-Dimethyl-4-acetyl-tetralin erhält man 1,1,4-Trimethyl-4-acetyl-tetralin, Sdp. 112°C/5 mmHg, $n_D^{20}$ : 1,5350. Geruch: holzig, cedrig, ambrig.

15 bis) 1,4-Dimethyl-4-propionyl-tetralin
Durch entsprechende Methylierung von 1-Methyl-4-propionyl-tetralin erhält man 1,4-Dimethyl-4-propionyl-tetralin, Sdp. 64°C/0,01 mmHg, $n_D^{20}$ : 1,5303. Geruch: fruchtig, aromatisch, grün, holzig, patchouli.

15 ter) 1,1,4,7-Tetramethyl-4-propionyl-tetralin
Durch entsprechende Methylierung von 1,1,7-Trimethyl-4-propionyl-tetralin erhält man 1,1,4,7-Tetramethyl-4-propionyl-tetralin, Sdp. 81°C/0,02 mmHg, $n_D^{20}$ : 1,5260. Geruch: holzig, Rosen, Himbeeren.

16) 1,1,3-Trimethyl-3-propionyl-indan
Durch entsprechende Methylierung von 1,1-Dimethyl-3-propionyl-indan erhält man 1,1,3-Trimethyl-3-propionyl-indan, Sdp. 63°C/0,01 mmHg, $n_D^{20}$ : 1,5150. Geruch: holzig, jononartig.

17) 1,1,3-Trimethyl-3-acetyl-indan
Durch entsprechende Methylierung von 1,1-Dimethyl-3-acetyl-indan erhält man 1,1,3-Trimethyl-3-acetyl-indan, Sdp. 88°C/3 mmHg, $n_D^{20}$ : 1,5158. Geruch: kamphrig, Patchouli.

Beispiel 2

In den folgenden Formulierungen steht U für 1,1-Dimethyl-4-propionyl-tetralin

1. Holzbase

| A: | Gewichtsteile |
|---|---|
| Basilikumöl | 30 |
| Methyljonon | 50 |
| p-tert.-Butylcyclohexylacetat | 50 |
| Methyldihydrojasmonat | 70 |
| Cedrylacetat krist. | 100 |
| Sandelholzöl | 200 |
| Patchouliöl | 200 |
| Bergamotteöl | 200 |
| | 900 |

B: wie oben, aber mit Zusatz von 100 Gewichtsteilen U.

Olfaktorische Beurteilung:

B wirkt angenehmer und frischer als A, die Bergamotte- Note tritt stärker hervor.

Nach 24 bzw. 48 Stunden wirkt A auf dem Riechstreifen staubig und wird dominiert vom Patchouliöl, wogegen B seinen Edelholzcharakter bewahrt hat und immer noch zedrig bzw. sandelholzartig riecht.

2. Chypre-Base:

| A: | Gewichtsteile |
|---|---|
| Styrallylacetat | 20 |
| Methylnonylacetaldehyd (10% in Diäthylphthalat) | 20 |
| Vetiverylacetat | 50 |
| Rhodinol | 50 |
| Patchouliöl | 50 |
| Baum-Moos absolut (50% in Diäthylphtalat) | 50 |
| p-tert.-Butyl-α-methylhydrozimtaldehyd | 100 |
| Hydroxycitronellal | 100 |
| Methyljonon | 100 |
| Ambrette-Moschus | 100 |
| Cumarin | 100 |
| Bergamotteöl | 100 |
| | 840 |

B: wie oben, aber mit Zusatz von 100 Gewichtsteilen U.

Olfaktorische Beurteilung:

A riecht zu stark nach Styrallacetat. In B dagegen wird die Bergamotte-Note vorteilhaft betont, die Base wirkt viel frischer. Nach 24 bzw. 48 Stunden wirkt B immer noch wesentlich frischer als A, wobei die jasminartige, blumige bzw. Aldehydische Note in B fixiert wird, in A hingegen verloren geht.

3. Grün-Base

| A: | Gewichtsteile |
|---|---|
| Citral | 10 |
| Wermutöl | 10 |
| Mastix absolut | 20 |
| Basilikumöl | 80 |
| Methyldihydrohjasmonat | 100 |
| Alkohol 95° | 130 |
| Linalylacetat | 200 |
| α-Hexylzimtaldehyd | 200 |
| Benzylsalicylat | 280 |
| | 950 |

B: wie oben, aber mit Zusatz von 50 Gewichtsteilen U.

Olfaktorische Beurteilung:
A wirkt hart und unausgeglichen; B dagegen weicher und harmonischer.
Diese Differenz tritt nach 24 bzw. 48 Stunden auf dem Riechstreifen noch viel deutlicher in Erscheinung. A wird von einer Wermutnote dominiert, und der Geruchseindruck endet mit einer staubigen Note, während in B ein Eindruck von grüner Frische viel länger erhalten bleibt.

4. Blumen-Base

| A: | Gewichtsteile |
|---|---|
| Laurin (Hydroxycitronellal extra) | 760 |
| Linalool | 70 |
| n-Hexylsalicylat | 30 |
| Cyclamenaldehyd | 20 |
| Galbanumöl | 20 |
| | 900 |

B: wie oben, aber mit Zusatz von 100 Gewichtsteilen U.

Olfaktorische Beurteilung:
A wirkt recht einseitig, Cyclamenaldehyd und das Galbanum treten zu stark hervor, was insbesondere beim 24 bzw. 48 Stunden-Wert beobachtet wird. B besticht durch einen vollen blumigen Charakter und bewahrt diesen über längere Zeit.

5. Herren-Cologne

| A: | Gewichtsteile |
|---|---|
| Bergamotteöl | 310 |
| Zitronenöl | 120 |
| α-Hexylzimtaldehyd | 100 |
| Methyldihydrojasmonat | 60 |
| α-Jonon | 60 |
| Basilikumöl | 50 |
| Rhodinol pur | 40 |
| Eugenol | 40 |
| Neroliöl | 40 |
| Patchouliöl | 20 |
| Baum-Moos abs. (50% in Propylen-glykol) | 20 |
| Vetivenylacetat | 20 |
| Ylang-Ylang-Oel | 20 |
| | 900 |

B: wie oben, aber mit Zusatz von 100 Gewichtsteilen U.

Olfaktorische Beurteilung:
B wirkt im Gegensatz zu A viel angenehmer und frischer, harmonisch und modern. Im Fond behält B seine Frische Citrus/Bergamotte-Note, während A bald stumpf und holzig wirkt.

6. Cologne classique

| A: | Gewichtsteile |
|---|---|
| Indol (10% in Carbitol) | 10 |
| Cumarin | 10 |
| Ylang-Ylang-C | 20 |
| Neroliöl | 40 |
| Lavandinöl | 40 |
| Benzylacetat | 40 |
| Eugenol extra | 40 |
| Citral | 60 |
| Methyldihydrojasmonat | 100 |
| α-Hexylzimtaldehyd | 100 |
| Zitronenöl italienisch | 100 |
| Bergamotteöl | 360 |
| | 920 |

B: wie oben, aber mit Zusatz von 80 Gewichtsteilen U.

Olfaktorische Beurteilung:

Das neue Tetralin unterstreicht die Citrusnote und verleiht der Komposition einen krautigen, lavenderlartigen Charakter, so dass B eindeutig bevorzugt ist.

Dank einer Neroli-noten-fixierung, die selbst nach 48 Stunden noch wahrnehmbar ist und in A völlig fehlt, wirkt B nach 24 Stunden immer noch reicher und süsser als A. Der Geruchskomplex in B wird durch das Tetralin zusammengehalten.

Beispiel 3

| Aprikosenaroma | Gewichtsteile A | Gewichtsteile B |
|---|---|---|
| Zimtaldehyd | 0,5 | 0,5 |
| Essigsäure-terpenylester | 1,25 | 1,25 |
| Anthranilsäure-methylester | 1,25 | 1,25 |
| Essigsäure-linalyester | 1,5 | 1,5 |
| Neroliöl bigarade | 2,5 | 2,5 |
| Geraniol | 5,0 | 5,0 |
| Buttersäure-amnylester | 8,0 | 8,0 |
| Essigsäure-i-amylester | 8,0 | 8,0 |
| Petitgrainöl Paraguay | 15,0 | 15,0 |
| Valeriansäure-amylester | 15,0 | 15,0 |
| Ameisensäure-amylester | 20,0 | 20,0 |
| Oenanthsäure-äthylester | 30,0 | 30,0 |
| α-Jonon | 30,0 | 10,0 |
| Vanillin | 80,0 | 80,0 |
| Benzaldehyd | 100,0 | 100,0 |
| Alkohol | 682,0 | 682,0 |
| U | | 20,0 |
| | 1000,0 | 1000,0 |

Durch den Zusatz des Tetralins U zu der Komposition A wird zwar die fruchtige Note etwas abgeschwächt. Es tritt aber zusätzlich (in B) eine leicht holzige Note in Erscheinung, die zusammen mit der fruchtigen Note stark an Trockenfrüchte (z.B. Aprikose) erinnert. Bei der Einarebeitung von B in Tabak ist die holzig-fruchtige Note ebenfalls deutlich feststellbar, die Tabaknote wird vorteilhaft abgerundet.

Das Aroma B kann als sog. topflavour für die Aromatisierung von Tabak (z. B. Zigaretten) Verwendung finden, die Richtdosierung beträgt z.B. 80—120, insb. 100 g/100 kg Tabak).

Beispiel 4

| Himbeeraroma | Gewichtsteile |
|---|---|
| α-Iron | 1 |
| Essigsäure-cis-3-hexenylester | 1 |
| Essigsäure-piperonylester | 4 |
| Vanillin | 5 |
| Essigsäure-äthylester | 5 |
| Aethylmaltol | 6 |
| Acetessigsäure-äthylester | 15 |
| Himbeerketon | 15 |
| Propylenglykol | 945 |
| | 997 |
| U | 3 |
| | 1000 |

Dosierung: z.B. 50 g auf 100 Liter Sirup

Durch den Zusatz von U zu obiger Komposition wird deren Geschmack deutlich voller und abgerundeter. Zusätzlich wird die für Himbeeraromen typische holzig-fruchtige Note vorteilhaft unterstrichen.

Geeignete Dosierung von U im Endprodukt: 1—3, insb. 1,5 ppm in Haushaltsirup; 2—4, insb. 3 ppm in Süsswaren.

## Patentansprüche

1. Riech- und/oder Geschmackstoffkompositionen, gekennzeichnet durch einen Gehalt an einer Verbindung der Formel

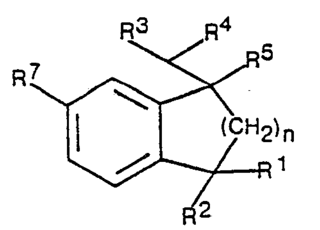

I

worin $R^1$—CO—$CH_2$—$R^6$ darstellt, die Reste $R^2$ bis $R^7$ für Wasserstoff oder methyl stehen und n = 1 oder 2 ist.

2. Riech- und/oder Geschmackstoffkompositionen, gekennzeichnet durch einen Gehalt an einer Verbindung der allgemeinen Formel

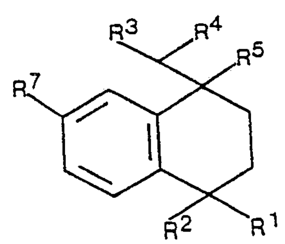

Ia

worin $R^1$ —CO—$CH_2$—$R^6$ darstellt und die Reste $R^2$ bis $R^7$ für Wasserstoff oder Methyl stehen.

3. Riech- und/oder Geschmackstoffkomposition, gekennzeichnet durch einen Gehalt an einer Verbindung der allgemeinen Formel

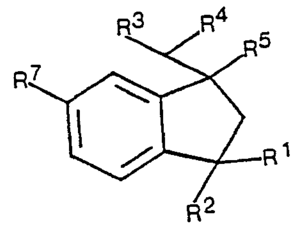

Ib

worin $R^1$ —CO—$CH_2$—$R^6$ darstellt und die Reste $R^2$ bis $R^7$ für Wasserstoff oder Methyl stehen.

4. Riech- und/oder Geschmackstoffkomposition, gekennzeichnet durch einen Gehalt an 1,1-Dimethyl-4-propionyltetralin.

5. Riech- und/oder Geschmackstoffkomposition, gekennzeichnet durch einen Gehalt an 1,1-Dimethyl-4-acetyl-tetralin.

6. Verwendung von Verbindungen der Formel

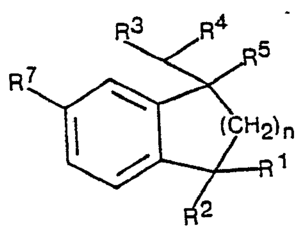

I

worin $R^1$ —CO—$CH_2$—$R^6$ darstellt, die Reste $R^2$ bis $R^7$ für Wasserstoff oder Methyl stehen und $n = 1$ oder 2 ist, als Riech- und/oder Geschmackstoffe.

7. Verbindungen der Formel

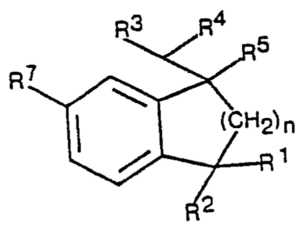

I

worin $R^1$ —CO—$CH_2$—$R^6$ darstellt, die Reste $R^2$ bis $R^7$ für Wasserstoff oder Methyl stehen und $n = 1$ oder 2 ist, mit der Ausnahme des 1,1-Dimethyl-3-acetylindans, des 1,1,3-Trimethyl-3-acetylindans und des 1,1,3,6-Tetramethyl-3-acetylindans.

8. Verbindungen der Formel

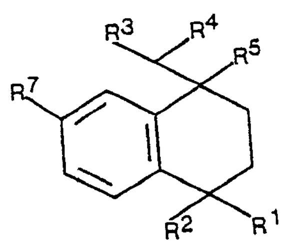

Ia

worin $R^1$ —CO—$CH_2$—$R^6$ darstellt, die Reste $R^2$ bis $R^7$ für Wasserstoff oder Methyl stehen.

9. 1,1-Dimethyl-4-propionyl-tetralin.

10. 1,1-Dimethyl-4-acetyl-tetralin.

11. Verbindungen der Formel

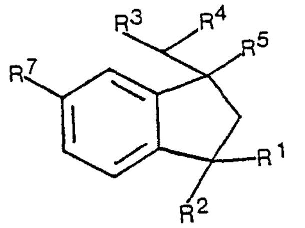

Ib

worin $R^1$ —CO—$CH_2$—$R^6$ darstellt, die Reste $R^2$ bis $R^7$ für Wasserstoff oder Methyl stehen, mit der Ausnahme des 1,1-Dimethyl-3-acetylindans, des 1,1,3-Trimethyl-3-acetylindans und des 1,1,3,6-Tetramethyl-3-acetylindans.

12. Eine Verbindung, ausgewählt unter 1-Methyl-4-propionyl-tetralin, 1,1,4-Trimethyl-4-acetyl-tetralin, 1,1,4-Trimethyl-4-propionyl-tetralin, 1,1,7-Trimethyl-4-propionyl-tetralin, 1,1,7-Trimethyl-4-acetyl-tetralin, 1-Methyl-4-acetyl-tetralin, 1,4-Dimethyl-4-propionyl-tetralin, 1,1,4,7-Tetramethyl-4-propionyl-tetralin, 1,1-Dimethyl-3-propionyl-indan, 1,1,3-Trimethyl-3-propionyl-indan.

13. Verbindungen der Formel

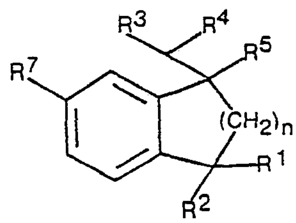

I

worin $R^1$ —CO—$CH_2$—$R^6$ darstellt, die Reste $R^2$ bis $R^7$ für Wasserstoff oder Methyl stehen und $n = 1$ oder 2 ist, als Riech- und/oder Geschmackstoffe.

**Revendications**

1. Compositions odorantes et/ou aromatisantes caractérisées en ce qu'elles contiennent un composé de formule

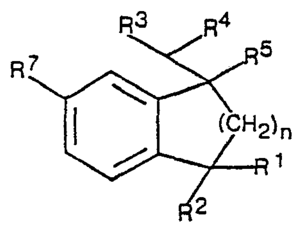

I

dans laquelle $R^1$ représente —CO—$CH_2$—$R^6$, les symboles $R^2$ à $R^7$ représentent l'hydrogène ou des groupes méthyle et n est égal à 1 ou 2.

2. Composition odorante et/ou aromatisante, caractérisée en ce qu'elle contient un composé de formule générale

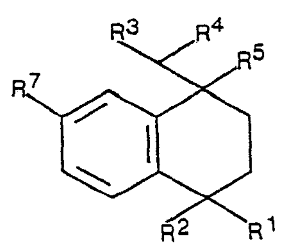

Ia

dans laquelle $R^1$ représente —CO—$CH_2$—$R^6$ et les symboles $R^2$ à $R^7$ représentent l'hydrogène ou des groupes méthyle.

3. Composition odorante et/ou aromatisante, caratérisée en ce qu'elle contient un composé de formule générale:

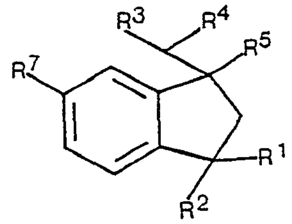

Ib

dans lequelle $R^1$ représente $—CO—CH_2—R^6$ et les symboles $R^2$ à $R^7$ représentent l'hydrogène ou des groupes méthyle.

4. Composition odorante et/ou aromatisante, caractérisée en ce qu'elle contient de la 1,1-diméthyl-4-propionyl-tétraline.

5. Composition odorante et/ou aromatisante, caractérisée en ce qu'elle contient de la 1,1-diméthyl-4-acétyl-tétraline.

6. Utilisation des composés de formule

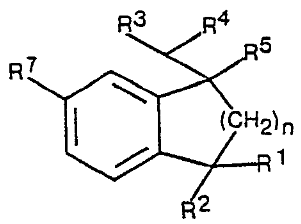

I

dans laquelle $R^1$ représente $—CO—CH_2—R^6$, les symboles $R^2$ à $R^7$ représentent l'hydrogène ou des groupes méthyle et n est égal à 1 ou 2, en tant que substances odorantes et/ou aromatisantes.

7. Composés de formule

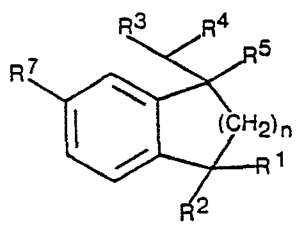

I

dans laquelle $R^1$ représente $—CO—CH_2—R^6$, les symboles $R^2$ à $R^7$ représentent l'hydrogène ou des groupes méthyle et n égal 1 ou 2, à l'exception du 1,1-diméthyl-3-acétylindane, du 1,1,3-triméthyl-3-acétylindane et du 1,1,3,6-tétraméthyl-3-acétylindane.

8. Composés de formule

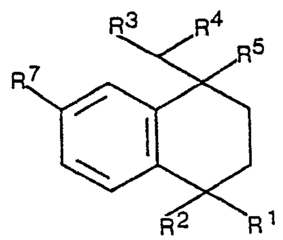

Ia

dans laquelle $R^1$ représente $—CO—CH_2—R^6$ et les symboles $R^2$ à $R^7$ représentent l'hydrogène ou des groupes méthyle.

9. La 1,1-diméthyl-4-propionyl-tétraline.

10. La 1,1-diméthyl-4-acétyl-tétraline.

11. Composés de formule

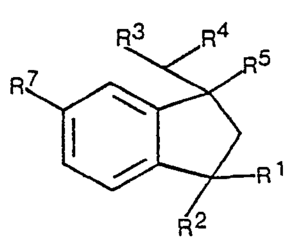

Ib

dans laquelle $R^1$ représente —CO—$CH_2$—$R^6$ et les symboles $R^2$ à $R^7$ représentent l'hydrogène ou des groupes méthyle, à l'exception du 1,1-diméthyl-3-acétylindane, du 1,1,3-triméthyl-3-acétylindane et du 1,1,3,6-tétraméthyl-3-acétylindane.

12. Un composé choisi parmi la 1-méthyl-4-propionyl-tétraline, la 1,1,4-triméthyl-4-acétyl-tétraline, la 1,1,4-triméthyl-4-propionyl-tétraline, la 1,1,7-triméthyl-4-propionyl-tétraline, la 1,1,7-triméthyl-4-acétyl-tétraline, la 1-méthyl-4-acétyl-tétraline, la 1,4-diméthyl-4-propionyl-tétraline, la 1,1,4,7-tétraméthyl-4-propionyl-tétraline, le 1,1,-diméthyl-3-propionyl-indane, le 1,1,3-triméthyl-3-propionyl-indane.

13. Composés de formule

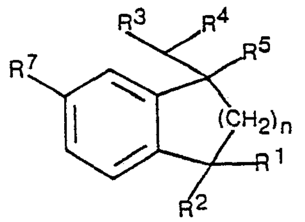

I

dans laquelle $R^1$ représente —CO—$CH_2$—$R^6$, les symboles $R^2$ à $R^7$ représentent l'hydrogène ou des groupes méthyle et n égal 1 ou 2, en tant que substances odorantes et/ou aromatisantes.

**Claims**

1. Odorant and/or flavouring substance compositions, characterized by a content of a compound of the formula

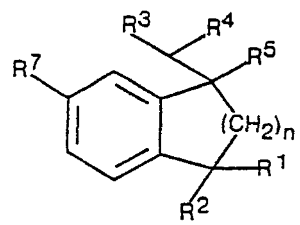

I

wherein $R^1$ represents —CO—$CH_2$—$R^6$, the residues $R^2$ to $R^7$ stand for hydrogen or methyl and $n = 1$ or 2.

2. Odorant and/or flavouring substance composition, characterized by a content of a compound of the general formula

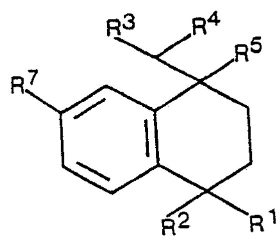

Ia

wherein $R^1$ represents —CO—$CH_2$—$R^6$ and the residues $R^2$ to $R^7$ stand for hydrogen or methyl.

3. Odorant and/or flavouring substance composition, characterized by a content of a compound of the general formula

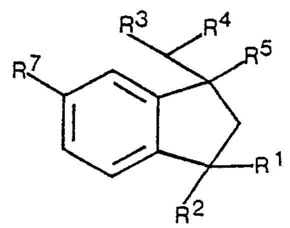

Ib

wherein $R^1$ represents —CO—$CH_2R^6$ and the residues $R^2$ to $R^7$ stand for hydrogen or methyl.

4. Odorant and/or flavouring substance composition, characterized by a content of 1,1-dimethyl-4-propionyltetralin.

5. Odorant and/or flavouring substance composition, characterized by a content of 1,1-dimethyl-4-acetyl-tetralin.

6. Use of compounds of the formula

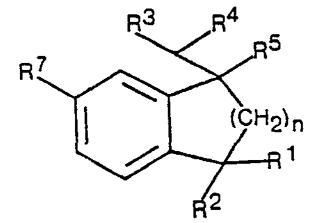

I

wherein $R^1$ represents —CO—$CH_2$—$R^6$ and the residues $R^2$ to $R^7$ stand for hydrogen or methyl and n = 1 or 2, as odorant and/or flavouring substances.

7. Compounds of the formula

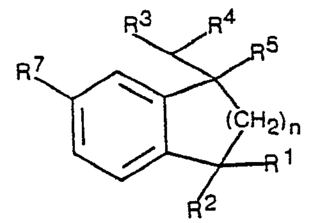

I

wherein $R^1$ represents —CO—$CH_2$—$R^6$ and the residues $R^2$ to $R^7$ stand for hydrogen or methyl and n = 1 or 2, with the exception of 1,1-dimethyl-3-acetylindane, of 1,1,3-trimethyl-3-acetylindane and of 1,1,3,6-tetramethyl-3-acetylindane.

8. Compounds of the formula

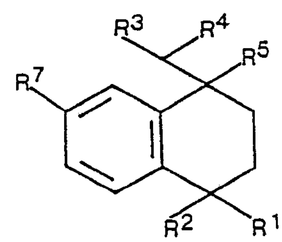

Ia

wherein $R^1$ represents —CO—$CH_2$—$R^6$ and the residues $R^2$ to $R^7$ stand for hydrogen or methyl.

9. 1,1-Dimethyl-4-propionyl-tetralin.

10. 1,1-Dimethyl-4-acetyl-tetralin.

11. Compounds of the formula

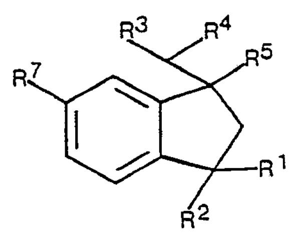

Ib

wherein $R^1$ represents —CO—$CH_2$—$R^6$ and the residues $R^2$ to $R^7$ stand for hydrogen or methyl, with the exception of 1,1-dimethyl-3-acetylindane, of 1,1,3-trimethyl-3-acetylindane and of 1,1,3,6-tetramethyl-3-acetylindane.

12. A compound selected from 1-methyl-4-propionyl-tetralin, 1,1,4-trimethyl-4-acetyl-tetralin, 1,1,4-trimethyl-4-propionyl-tetralin, 1,1,7-trimethyl-4-propionyl-tetralin, 1,1,7-trimethyl-4-acetyl-tetralin, 1-methyl-4-acetyl-tetralin, 1,4-dimethyl-4-propionyl-tetralin, 1,1,4,7-tetramethyl-4-propionyl-tetralin, 1,1-dimethyl-3-propionyl-indane, 1,1,3-trimethyl-3-propionyl-indane.

13. Compounds of the formula

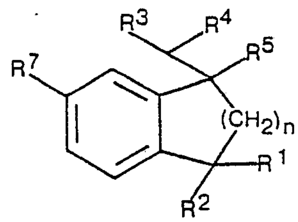

wherein $R^1$ represents $-CO-CH_2-R^6$, the residues $R^2$ to $R^7$ stand for hydrogen or methyl and n = 1 or 2, as odorant and/or flavouring substances.